Europäisches Patentamt

European Patent Office

Office européen des brevets

(19)

(11) Veröffentlichungsnummer: **0 141 079 B1**

(12) **EUROPÄISCHE PATENTSCHRIFT**

(45) Veröffentlichungstag der Patentschrift: **08.01.92**

(21) Anmeldenummer: **84109406.3**

(22) Anmeldetag: **08.08.84**

(51) Int. Cl.⁵: **C12P 21/08**, C07K 15/02, G01N 33/577, A61K 47/00

(54) Monoklonale Antikörper mit Spezifität für membran-assoziierte Antigene.

(30) Priorität: **12.08.83 DE 3329184**

(43) Veröffentlichungstag der Anmeldung:
**15.05.85 Patentblatt 85/20**

(45) Bekanntmachung des Hinweises auf die Patenterteilung:
**08.01.92 Patentblatt 92/02**

(84) Benannte Vertragsstaaten:
**AT BE CH DE FR GB IT LI LU NL SE**

(56) Entgegenhaltungen:
**EP-A- 0 119 556**
**EP-A- 0 125 928**
**WO-A-81/01469**

**LA RICERCA CLIN. LAB. Band 12, 1982, Seiten 517-538; B.S. WILSON et al.: "Human melanoma-associated antigens identified with monoclonal antibodies"**

(73) Patentinhaber: **BEHRINGWERKE Aktiengesellschaft**
**Postfach 1140**
**W-3550 Marburg 1(DE)**

(72) Erfinder: **Bosslet, Klaus, Dr.**
**Ockershäuser Allee 5**
**W-3550 Marburg 1(DE)**
Erfinder: **Kurrle, Roland, Dr.**
**Kiefernweg 12**
**W-3556 Niederweimar(DE)**
Erfinder: **Sedlacek, Hans-Harald, Dr.**
**Am Sonnenhang 3**
**W-3550 Marburg 1(DE)**
Erfinder: **Kanzy, Ernst-Jürgen**
**Huteweg 3**
**W-3556 Weimar-Niederweimar(DE)**
Erfinder: **Katoh, Takako, Dr.**
**3-35-10-Higashi**
**Chishi Rodai Shiba 280(JP)**

BIOLOGICAL ABSTRACTS, Band 73, Heft 1, 1982, Seite 431, Spalte 2, Zusammenfassung Nr. 4132, Phila., PA, US; S.M. LOOP et al.: "Human tumor-associated antigens, p155 and p210, detected by monoclonal antibodies" & INT. J. CANCER 27(6): 775-782, 1981

INT. J. CANCER, Band 28, 1981, Seiten 293-300; B.S. WILSON et al.: "Distribution and molecular characterization of a cell-surface and a cytoplasmic antigen detectable in human melanoma cells with monoclonal antibodies"

EUR. JOURNAL IMMUNOLOGY, Band 11, 1981, Seiten 825-831, Verlag Chemie GmbH, Weinheim, DE; J.P. JOHNSON et al.: "Surface antigens of human melanoma cells defined by monoclonal antibodies. I. Biochemical characterization of two antigens found on cell lines and fresh tumors of diverse tissue origin"

J. EXP. MED., Band 156, December 1982, Seiten 1755-1766, The Rockefeller University Press; A.N. HOUGHTON et al.: "Surface antigens of melanocytes and melanomas"

(74) Vertreter: Becker, Heinrich Karl Engelbert, Dr. et al
HOECHST AKTIENGESELLSCHAFT Central Patent Department P.O. Box 80 03 20
W-6230 Frankfurt am Main 80(DE)

## Beschreibung

Die Erfindung betrifft monoklonale Antikörper, die an definierte membran-assoziierte Antigene binden. Diese Antikörper können als Diagnostikum oder als Wirkstoffträger verwendet werden.

Die Technik der Immunisierung mit definierten, isolierten Antigenen und der Produktion von Antikörpern gegen solche Antigene ist bekannt. Es ist auch bekannt, mit ungereinigtem Antigenmaterial zu immunisieren und diejenigen Antikörper zu selektieren, die eine bestimmte Komponente einer solchen Antigenmischung erkennen.

Bei dem Versuch, solche Antikörper zu induzieren, ist es gelungen, monoklonale Antikörper zu selektieren, die unter nicht reduzierenden Bedingungen mit folgenden Proteinantigenen reagieren: Antigen 1: MW 33 KD ± 3, Antigen 2: MW 134 KD ± 3, Antigen 3: MW 80 KD ± 3, Antigen 4: 55 KD ± 3, Antigen 5: 60 KD ± 3, Antigen 6: 54 KD ± 3, Antigen 7: 260 KD ± 3, Antigen 8: nicht bestimmbar, Antigen 9: nicht bestimmbar, Antigen 10: MW 143 KD ± 3, 119 KD ± 3, Antigen 11: MW 178 KD ± 3, Antigen 12: MW nicht bestimmbar, Antigen 13: MW 34 KD ± 3, Antigen 14: MW 195 KD ± 3, Antigen 15: MW 44 KD ± 7, Antigen 16: MW 43 KD ± 3, Antigen 17: MW 130 KD ± 3.

Die auf den Antigenen 1 - 17 von den AK 1 - 17 erkannten Epitope sind gegen eine Behandlung mit Dithiothreitol (50 mmol/l, 2 Std., 37° C) unterschiedlich empfindlich: Die Epitope von Ag1, 2 und 15 werden durch obige Behandlung verändert, die von Ag4, 10 und 14 bleiben unverändert.

Die in vitro Spezifitäten der AK 1 - 17 sind in Tabelle I dargestellt.

Gegenstand der Erfindung sind monoklonale Antikörper, die ein Antigen (Ag1) erkennen mit einem Molekulargewicht von annähernd 33 KD ± 3 oder ein

Antigen (Ag2) mit einem Molekulargewicht von annähernd 134 KD ± 3 oder ein

Antigen (Ag3) mit einem Molekulargewicht von annähernd 80 KD ± 3 oder ein

Antigen (Ag4) mit einem Molekulargewicht von annähernd 55 KD ± 3 oder ein

Antigen (Ag5) mit einem Molekulargewicht von annähernd 60 KD ± 3 oder ein

Antigen (Ag6) mit einem Molekulargewicht von annähernd 54 KD ± 3 oder ein

Antigen (Ag7) mit einem Molekulargewicht von annähernd 260 KD ± 3 oder ein

Antigen (Ag8) mit einem nicht bestimmbaren Molekulargewicht oder ein

Antigen (Ag9) mit einem nicht bestimmbaren Molekulargewicht oder ein

Antigen (Ag10) mit einem Molekulargewicht von annähernd 143 KD ± 3, und 119 KD ± 3 oder ein

Antigen (Ag11) mit einem Molekulargewicht von annähernd 178 KD ± 3 oder ein

Antigen (Ag12) mit einem nicht bestimmbaren Molekulargewicht oder ein

Antigen (Ag13) mit einem Molekulargewicht von annähernd 34 KD ± 3 oder ein

Antigen (Ag14) mit einem Molekulargewicht von annähernd 195 KD ± 3 oder ein

Antigen (Ag15) mit einem Molekulargewicht von annähernd 44 KD ± 7 oder ein

Antigen (Ag16) mit einem Molekulargewicht von annähernd 43 KD ± 3 oder ein Antigen (Ag17) mit einem Molekulargewicht von annähernd 130 KD ± 3.

Die Antigene 4,8,9,10 sind Mycoplasmenantigene, die auf in der Tabelle beschriebenen Zellinien vorhanden sind.

Als Immunogen zur Induktion dieser Antikörper dienen menschliche in vitro-kultivierte Zellinien, Zellextrakte oder Extrakte aus humanen Geweben. Bevorzugt werden permanente humane Zellinien, in besonderem die CaLu-1, Chago, Oat 75, PaTu II und Bewo-Zellinie. Es kann auch Ag1 - Ag17 benutzt werden um AK1 - AK17 zu erzeugen.

Säugetiere, vorzugsweise Mäuse, werden mit 1 x 10⁶ - 10⁸ Zellen, vorzugsweise aber 10⁷ Zellen einer solchen Zellinie intraperitoneal immunisiert (Tag 0 - 120, vorzugsweise aber an Tag 0 und 7) und nach 1 - 150 Tagen, vorzugsweise aber an Tag 11, die Milzzellen aus solchen Tieren mit der X63 Ag 8653 Zellinie (The Journal of Immunology 173, 4, 1548-1550, 1979) fusioniert (Nature 256, 495-497, 1975).

Die nach 3 Wochen entstehenden Hybridome werden getestet, ob sie Antikörper der gewünschten Spezifität enthalten. In diesem Fall wurde eine Palette von 30 in vitro-kultivierten Zellinien, humane periphere Blutzellen und menschliches Knochenmark mit Hilfe von indirekter Immunfluoreszens (Behring Inst. Mitt. 59, 64-70, 1976) und Zellsorter-Analyse (Acta Cytol. 19, 374-377, 1975),(Proc. Natl. Acad. Sci. USA 77/8, 4914-4917,1980) auf Reaktivität mit den Antikörpern getestet.

Überraschenderweise befanden sich unter den ausgetesteten Hybridüberständen einige, die Antikörper der oben beschriebenen interessanten Spezifitäten enthielten. Die diese Antikörper sezernierenden Hybridome wurden mittels Mikromanipulator kloniert und die von diesen Hybridomaklonen gewonnenen monoklonalen Antikörper benutzt, um das von ihnen erkannte Antigen immunchemisch zu charakterisieren. Hierzu werden Zellgeister (Hybridoma, 1, 4, 413-421 (1982)), die aus Gewebekulturzellen gewonnen werden, mittels Detergens solubilisiert, ultrazentrifugiert und die so gewonnene Antigenmischung unter nicht reduzierenden Bedingungen im SDS-Polyacrylamidgel (10 g/100 ml Acrylamid / 0.026 g/100 ml Bisacrylamid elektrophoretisch aufgetrennt (Nature, 127, 680-685 (1970)). Danach werden alle so getrennten Antigene mittels Elektroblotting vom SDS-Gel auf Nitrocellulosefilter übertragen. (Proc. Natl. Acad. Sci. USA, 76, 4350-4354 (1979)). Anschließend erfolgt die Reaktion der auf dem Nitrocellulosefilter immobilisierten Antigenen mit dem spezifischen Antikörper (Hoppe-Seylers Z. Physiol. Chem. 363, 1133-1140,(1982))-Der Nachweis der Bindung dieses Antikörpers wird durch Reaktion mit $^{125}$J-Protein A und Autoradiographie des Filters erbracht. Das Molekulargewicht wird bestimmt im Vergleich zu kommerziell erhältlichen Markern. Eine präparative Reinigung der entsprechenden Antigene erfolgt affinitätschromatographisch.

Nitrocellulosepapier (15 x 15 cm) (Hoppe-Seylers Z. Physiol. Chem. 363, 1133-1140, (1982)) wird nach Befeuchten mittels phosphatgepufferter Kochsalzlösung (PBS pH 7,2)mit gereinigtem monoklonalem Antikörper-Protein inkubiert (1 Std., 4°C, 30 ml, 1-100 μg Protein/ml). Danach wird das ungebundene Protein durch Waschen in 500 ml PBS entfernt. Nach Inkubation des Filters für 1 Std. bei 40°C in 3 g/100 ml BSA (Ochsenserumalbumin) 0,05 g/100 ml Tween 20 in PBS pH 7,2 (Blockierung) wird das Filter nach dreimaligem Waschen mit PBS mit ungereinigter Zellextrakten, die in 0,5 % Natriumdeoxycholate, 20 mM Phenylmethylsulfonylfluorid in PBS gelöst sind, für 1 Std. bei 4°C inkubiert.

Anschließend wird das nicht gebundene Material mittels 3maligem Waschen mit PBS entfernt. Das spezifisch gebundene Antigen wird durch Inkubation des Filters in 1 - 9 M NH$_4$SCN bevorzugt 6 M NH$_4$SCN in PBS für 5 bis 30 Minuten bevorzugt 15 Minuten bei 4°C abgelöst.

Das so gereinigte Antigen kann nach Entfernung des NH$_4$SCN (gelchromatographisch, Dialyse) als Immunogen bzw. für andere Zwecke Verwendung finden.

Die aufgrund ihrer Reaktivität mit Ag1 und Ag2 charakterisierten monoklonalen Antikörper können zur Unterscheidung von aus solidem menschlichem Gewebe abgeleiteten Zellen und von tierischen Zellen als in vitro-Diagnostikum eingesetzt werden. Weiterhin könnten sie als Positiv-Kontrolle für menschliches Gewebe in der Immunhistologie und zur Unterscheidung von tierischem Gewebe Verwendung finden.

AK1 bis AK 17 können weiterhin dazu verwendet werden, verbunden mit Wirkstoffen gezielt zum Wirkort zu bringen, z. B. bei der autologen Knochenmarkstransplantation. Die Verwendung dieser Antikörper zum Nachweis der entsprechenden Ag in Körperflüssigkeiten, stellt eine diagnostische Anwendungsmöglichkeit dieser Ak dar. Die Ak können in Konzentrationen von 0.01 μg bis 1 mg/ml verwendet werden.

## Reaktivität von AK 1 - 17 auf in vitro-Zellen

| Getestete Zellen | 1 | 2 | 3 | 4 | 5 | 6 | 7 | 8 | 9 | 10 | 11 | 12 | 13 | 14 | 15 | 16 | 17 |
|---|---|---|---|---|---|---|---|---|---|---|---|---|---|---|---|---|---|
| **Lungentumorzell-linien** | | | | | | | | | | | | | | | | | |
| CaLu-1 | + | + | | + | | | | + | + | + | + | + | + | | | − | |
| E-14 | + | − | − | + | + | + | + | + | + | + | + | − | − | − | − | − | + |
| B 109/4 | + | + | − | + | | | | − | − | − | | − | | | − | − | − |
| A 549 | + | + | | + | | | | + | − | − | − | − | | | | − | |
| Oat 75 | + | + | − | + | − | + | + | + | − | + | − | − | − | + | + | − | + |
| SHP-77 | + | + | − | + | | | | + | + | + | | − | + | | | − | |
| Chago | + | − | − | + | + | − | − | | + | + | − | − | − | − | − | − | + |
| Bro-Ca-Hoff | + | + | − | − | + | | − | | − | | + | − | − | | | − | |
| **Pankreastumor-zellinien** | | | | | | | | | | | | | | | | | |
| Pa Tu II | + | + | − | − | − | − | + | − | − | − | − | + | − | − | − | + | ± |
| Pa Tu III | | + | − | + | | − | − | + | − | + | | + | − | | − | − | |
| MIA-Pa-Ca 2 | + | + | − | − | − | + | − | − | − | | − | − | | | − | − | |
| Panc-1 | + | + | − | + | − | + | + | − | | − | − | − | − | | − | − | |
| **Gynäkologische Tumorzellinien** | | | | | | | | | | | | | | | | | |
| Bewo | + | − | + | + | + | + | + | | − | | | − | − | + | + | − | + |
| HeLa | + | + | | + | − | + | + | − | − | | − | − | − | + | + | − | + |
| Pa-1 | + | ÷ | + | + | − | + | − | − | + | − | | − | − | | − | − | + |
| **Melanomzell-linien** | | | | | | | | | | | | | | | | | |
| Mel-ULF | + | + | − | + | − | + | + | − | ± | | − | + | − | + | − | + | + |
| Mel-RPMI | + | + | − | + | − | + | + | − | + | | − | − | − | + | − | + | |
| Mel-51-2 | + | + | − | + | − | − | − | − | ± | | − | − | − | − | − | − | + |
| Mel-21-C-48 | + | + | − | + | + | − | + | − | + | | + | − | + | + | − | + | + |
| **nicht verwandte Tumorzellinien** | | | | | | | | | | | | | | | | | |
| ZR-75-1 | + | + | − | | − | + | + | − | | | − | | | + | − | | − |
| MCF-7 | + | + | − | + | | − | − | − | | − | | − | | − | − | − | − |
| Mamma-Ca-12 | + | | − | | − | + | + | | | | | | | + | − | | |
| Colon-Ca-Ax | + | | | − | | | | − | | − | | − | | | − | | |

| Getestete Zellen | 1 | 2 | 3 | 4 | 5 | 6 | 7 | 8 | 9 | 10 | 11 | 12 | 13 | 14 | 15 | 16 | 17 |
|---|---|---|---|---|---|---|---|---|---|---|---|---|---|---|---|---|---|
| Colon-Wi | + | + | − | − | − | − | − |  | − | − |  | − |  | − | − | − |  | − |
| HT-1080 | + |  |  |  |  |  |  |  |  |  | + |  | − |  |  |  |  |
| Leiomyosarkom | + |  | + |  |  |  |  |  |  |  |  |  |  |  |  |  |  |
| Hypernephrom TUW |  |  |  |  |  |  |  |  |  |  |  |  | − |  |  |  |  |
| IMR 32 | + | − | − |  | − | − |  | − | − | − |  |  | − | − | − |  | − |
| Raji | + |  | − |  | − | − | − |  | − |  | − |  | − | − | − |  |  |
| Daudi | + |  | − |  | − | − | − |  | − |  | − |  | − | − | − |  |  |
| 1788 | + |  | − |  | − | − | − |  | − |  | − |  |  |  |  |  |  |
| 6666/1 | + |  | − | − | − |  | − |  | − |  |  | − | − | − |  |  |  |
| Immunozytom | + |  | − |  | − | + | − |  |  |  |  |  |  | − | − |  |  |

**Normale lymphoide Zellen**

| | 1 | 2 | 3 | 4 | 5 | 6 | 7 | 8 | 9 | 10 | 11 | 12 | 13 | 14 | 15 | 16 | 17 |
|---|---|---|---|---|---|---|---|---|---|---|---|---|---|---|---|---|---|
| Lymphozyten | + | + | − | − | − | − | − | − | − | − |  | ± | − | − | − | ± | − |
| Monozyten | + | + | − | − | − | − | − | − | − | − |  | − | − | − | − | + | − |
| Granulozyten | + | + | − | − | + | − | − | − | − |  |  | − | − | − | − | − | ± |
| Erythrozyten | − | − | − | − | − | − | − | − |  | − |  | − | − | − | − | − | − |
| Knochenmark | + | + | − | − | + | − | − | − | − |  |  | − | − | − | − | + |  |

**Normale humane Fibroblasten**

| | 1 | 2 | 3 | 4 | 5 | 6 | 7 | 8 | 9 | 10 | 11 | 12 | 13 | 14 | 15 | 16 | 17 |
|---|---|---|---|---|---|---|---|---|---|---|---|---|---|---|---|---|---|
| LL-29 | + | + |  | − | − |  | − | − | − |  |  | − | − |  | − | − | − |
| Hu-Fi-Br 32 | + | + | + | − | − | − | − | − | − |  |  | − | − | − |  | − | − |
| Hu-Fi-Br 43 | + | + |  | − | − |  | − | − | − | − |  | − | − |  | − | − |  |
| Hu-Fi-Br 47 | + | + |  | − | − |  | − | − | − | − |  | − | − |  | − |  |  |
| Hu-Fi-Br 16 | + | + | + | − | − | − |  | − | − | − |  | − | − | − | − | − |  |
| Hu-Fi-Mel-1 | + | + | + | − | − | − | − | − |  | − |  | − | − |  | − | − |  |
| Hu-Fi-Mag-13 | + | + |  | − | − | − | − | − | − |  |  | − | − | − |  | − | − |

**Tierische Zellen**

| | 1 | 2 | 3 | 4 | 5 | 6 | 7 | 8 | 9 | 10 | 11 | 12 | 13 | 14 | 15 | 16 | 17 |
|---|---|---|---|---|---|---|---|---|---|---|---|---|---|---|---|---|---|
| Vero | − | + | − | − | − | − | − |  | − | − |  | − | − |  | − | + | − |
| Greyhound | − | − | − | − | − | − | − | − | − |  |  | − | − | − | − | − |  |
| BHK | − | − | − | − | − | − | + | − | − |  |  | − | − | − | − | − |  |
| Rattenfibroblasten | − | − | − | − | − | − | − | − | − |  |  | − | − | − | − | − |  |
| Mausfibroblasten | − | − | − | − | − | − | − | − | − |  |  | − | − | − | − | − |  |

EP 0 141 079 B1

+ = signifikant positive Reaktion im indirekten Immunfluoreszenztest, im Radioimmunoassay und in der
cytofluorometrischen Analyse.

± = schwache positiv Reaktion mit einem Teil der getesteten Population

– = keine signifikante Reaktion

**Patentansprüche**

1. Monoklonale Antikörper, die ein Antigen (Ag1) mit einem Molekulargewicht von annähernd 33 KD ± 3, ein Antigen (Ag2) mit einem Molekulargewicht von annähernd 134 KD ± 3, ein Antigen (Ag3) mit einem Molekulargewicht von annähernd 80 KD ± 3, ein Antigen (Ag4) mit einem Molekulargewicht von annähernd 55 KD ± 3, ein Antigen (Ag5) mit einem Molekulargewicht von annähernd 60 KD ± 3, ein Antigen (Ag6) mit einem Molekulargewicht von annähernd 54KD ± 3, ein Antigen (Ag7) mit einem Molekulargewicht von annähernd 260 KD ± 3, ein Antigen (Ag8) mit einem nicht bestimmbaren Molekulargewicht, ein Antigen (Ag9) mit einem nicht bestimmbaren Molekulargewicht, ein Antigen (Ag10) mit einem Molekulargewicht von annähernd 143 KD ± 3 und 119 KD ± 3, ein Antigen (Ag11) mit einem Molekulargewicht von annähernd 178 KD ± 3, ein Antigen (Ag12) mit einem nicht bestimmbaren Molekulargewicht, ein Antigen (Ag13) mit einem Molekulargewicht von annähernd 34 KD ± 3, ein Antigen (Ag14) mit einem Molekulargewicht von annähernd 195 KD ± 3, ein Antigen (Ag15) mit einem Molekulargewicht von annähernd 44 KD ± 7, ein Antigen (Ag16) mit einem Molekulargewicht von annähernd 43 KD ± 3 oder ein Antigen (Ag17) mit einem Molekulargewicht von 130 KD ± 3 erkennen, die mit den in Tabelle I aufgeführten Zellinien die spezifischen Reaktionsmuster zeigen und die durch Immunisierung von Säugetieren mit menschlichen in vitro - kultivierten Zellinien, Zellextrakten oder Extrakten aus humanen Geweben hergestellt werden.

2. Antigen, gekennzeichnet durch ein Molekulargewicht von 33 KD ± 3 (Ag1) oder 134 KD ± 3 (Ag2), ein Antigen (Ag3) mit einem Molekulargewicht von annähernd 80 KD ± 3, ein Antigen (Ag4) mit einem Molekulargewicht von annähernd 55 KD ± 3, ein Antigen (Ag5) mit einem Molekulargewicht von annähernd 60 KD ± 3, ein Antigen (Ag6) mit einem Molekulargewicht von annähernd 54 KD ± 3, ein Antigen (Ag7) mit einem Molekulargewicht von annähernd 260 KD ± 3, ein Antigen (Ag8) mit einem nicht bestimmbaren Molekulargewicht, ein Antigen (Ag9) mit einem nicht bestimmbaren Molekulargewicht, ein Antigen (Ag10) mit einem Molekulargewicht von annähernd 143 KD ± 3 und 119 KD ± 3, ein Antigen (Ag11) mit einem Molekulargewicht von annähernd 178 KD ± 3, ein Antigen (Ag12) mit einem nicht bestimmbaren Molekulargewicht, ein Antigen (Ag13) mit einem Molekulargewicht von annähernd 34 KD ± 3, ein Antigen (Ag14) mit einem Molekulargewicht von annähernd 195 KD ± 3, ein Antigen (Ag15) mit einem Molekulargewicht von annähernd 44 KD ± 7 oder ein Antigen (Ag16) Mit einem Molekulargewicht von annähernd 43 ± 3 und ein Antigen (Ag17) mit einem Molekulargewicht von 130 KD ± 3 und der in Tabelle angegebenen Verteilung auf menschlichen und tierischen Zellen.

3. Verfahren zur Herstellung eines monoklonalen Antikörpers, nach Anspruch 1, dadurch gekennzeichnet, daß Säugetiere mit Zellen einer menschlichen in vitro-kultivierten Zellinie, eines Zellextraktes aus einer solchen Zellinie oder einem Extrakt aus einem humanen Gewebe immunisiert werden, Milzzellen aus einem solchen immunisierten Tier entnommen, mit der Zellinie X 63 Ag 8653 fusioniert, die Hybridome selektioniert und der monoklonale Antikörper gewonnen wird.

4. Verfahren nach Anspruch 2, dadurch gekennzeichnet, daß die menschliche in vitro-kultivierte Zellinie eine permanente Zellinie ist.

5. Verfahren nach Anspruch 2, dadurch gekennzeichnet, daß die Säugetiere Mäuse sind.

7

6. Verwendung eines monoklonalen Antikörpers nach Anspruch 1 in einem in vitro-Diagnostikum.

7. Verwendung eines monoklonalen Antikörpers nach Anspruch 1 als Träger für einen pharmazeutischen Wirkstoff.

**Claims**

1. Monoclonal antibodies which recognize an antigen (Ag1) having a molecular weight of approximately 33 KD ± 3, an antigen (Ag2) having a molecular weight of approximately 134 KD ± 3, an antigen (Ag3) having a molecular weight of approximately 80 KD ± 3, an antigen (Ag4) having a molecular weight of approximately 55 KD ± 3, an antigen (Ag5) having a molecular weight of approximately 60 KD ± 3, an antigen (Ag6) having a molecular weight of approximately 54 KD ± 3, an antigen (Ag7) having a molecular weight of approximately 260 KD ± 3, an antigen (Ag8) having a non-determinable molecular weight, an antigen (Ag9) having a non-determinable molecular weight, an antigen (Ag10) having a molecular weight of approximately 143 KD ± 3 and 119 KD ± 3, an antigen (Ag11) having a molecular weight of approximately 178 KD ± 3, an antigen (Ag12) having a non-determinable molecular weight, an antigen (Ag13) having a molecular weight of approximately 34 KD ± 3, an antigen (Ag14) having a molecular weight of approximately 195 KD ± 3, an antigen (Ag15) having a molecular weight of approximately 44 KD ± 7, an antigen (Ag16) having a molecular weight of approximately 43 KD ± 3 or an antigen (Ag17) having a molecular weight of 130 KD ± 3, which have the specific reaction patterns with the cell lines listed in Table I and which are prepared by immunization of mammals with human in vitro cultivated cell lines, cell extracts or extracts from human tissues.

2. An antigen which has a molecular weight of 33 KD ± 3 (Ag1) or 134 KD ± 3 (Ag2), an antigen (Ag3) having a molecular weight of approximately 80 KD ± 3, an antigen (Ag4) having a molecular weight of approximately 55 KD ± 3, an antigen (Ag5) having a molecular weight of approximately 60 KD ± 3, an antigen (Ag6) having a molecular weight of approximately 54 KD ± 3, an antigen (Ag7) having a molecular weight of approximately 260 KD ± 3, an antigen (Ag8) having a non-determinable molecular weight, an antigen (Ag9) having a non-determinable molecular weight, an antigen (Ag10) having a molecular weight of approximately 143 KD ± 3 and 119 KD ± 3, an antigen (Ag11) having a molecular weight of approximately 178 KD ± 3, an antigen (Ag12) having a non-determinable molecular weight, an antigen (Ag13) having a molecular weight of approximately 34 KD ± 3, an antigen (Ag14) having a molecular weight of approximately 195 KD ± 3, an antigen (Ag15) having a molecular weight of approximately 44 KD ± 7, or an antigen (Ag16) having a molecular weight of approximately 43 KD ± 3, and an antigen (Ag17) having a molecular weight of 130 KD ± 3 and the distribution over human and animal cells indicated in the table.

3. A process for the preparation of a monoclonal antibody as claimed in claim 1, which comprises immunizing mammals with cells of a human cell line cultivated in vitro, a cell extract from a cell line of this type or an extract from a human tissue, removing spleen cells from such an immunized animal, fusing them with the cell line X 63 Ag 8653, selecting the hybridomas, and obtaining the monoclonal antibodies.

4. The process as claimed in claim 3, wherein the human cell line cultivated in vitro is a permanent cell line.

5. The process as claimed in claim 3, wherein the mammals are mice.

6. The use of a monoclonal antibody, as claimed in claim 1, in an in vitro diagnostic aid.

7. The use of a monoclonal antibody, as claimed in claim 1, as carrier for a pharmaceutical active compound.

**Revendications**

1. Anticorps monoclonaux qui reconnaissent un antigène (Ag1) ayant une masse moléculaire d'environ 33 kd ± 3, un antigène (Ag2) ayant une masse moléculaire d'environ 134 kd ± 3, un antigène (Ag3) ayant une masse moléculaire d'environ 80 kd ± 3, un antigène (Ag4) ayant une masse moléculaire d'environ 55 kd ± 3, un antigène (Ag5) ayant une masse moléculaire d'environ 60 kd ± 3, un antigène (Ag6) ayant une masse moléculaire d'environ 54 kd ± 3, un antigène (Ag7) ayant une masse moléculaire d'environ 260 kd ± 3, un antigène (Ag8) ayant une masse moléculaire non déterminable, un antigène (Ag9) ayant une masse moléculaire non déterminable, un antigène (Ag10) ayant une masse moléculaire d'environ 143 kd ± 3 et de 119 kd ± 3, un antigène (Ag11) ayant une masse moléculaire d'environ 178 kd ± 3, un antigène (Ag12) ayant une masse moléculaire non déterminable, un antigène (Ag13) ayant une masse moléculaire d'environ 34 kd ± 3, un antigène (Ag14) ayant une masse moléculaire d'environ 195 kd ± 3, un antigène (Ag15) ayant une masse moléculaire d'environ 44 kd ± 7, un antigène (Ag16) ayant une masse moléculaire d'environ 43 kd ± 3 ou un antigène (Ag17) ayant une masse moléculaire de 130 kd ± 3, qui présentent les types de réactions spécifiques avec les lignées cellulaires indiquées dans le tableau 1, et sont obtenus par immunisation de mammifères avec des lignées cellulaires humaines cultivées in vitro, des extraits cellulaires ou des extraits de tissus humains.

2. Antigène caractérisé par une masse moléculaire de 33 kd ± 3 (Ag1) ou de 134 kd ± 3 (Ag2), un antigène (Ag3) ayant une masse moléculaire d'environ 80 kd ± 3, un antigène (Ag4) ayant une masse moléculaire d'environ 55 kd ± 3, un antigène (Ag5) ayant une masse moléculaire d'environ 60 kd ± 3, un antigène (Ag6) ayant une masse moléculaire d'environ 54 kd ± 3, un antigène (Ag7) ayant une masse moléculaire d'environ 260 kd ± 3, un antigène (Ag8) ayant une masse moléculaire non déterminable, un antigène (Ag9) ayant une masse moléculaire non déterminable, un antigène (Ag10) ayant une masse moléculaire d'environ 143 kd ± 3 et de 119 kd ± 3, un antigène (Ag11) ayant une masse moléculaire d'environ 178 kd ± 3, un antigène (Ag12) ayant une masse moléculaire non déterminable, un antigène (Ag13) ayant une masse moléculaire d'environ 34 kd ± 3, un antigène (Ag14) ayant une masse moléculaire d'environ 195 kd ± 3, un antigène (Ag15) ayant une masse moléculaire d'environ 44 kd ± 7 ou un antigène (Ag16) ayant une masse moléculaire d'environ 43 kd ± 3 et un antigène (Ag17) ayant une masse moléculaire de 130 kd ± 3, et la distribution sur des cellules humaines et animales indiquée dans le tableau.

3. Procédé pour la production d'un anticorps monoclonal selon la revendication 1, caractérisé en ce que l'on immunise des mammifères avec des cellules d'une lignée cellulaire humaine cultivée in vitro, d'un extrait cellulaire provenant d'une telle lignée cellulaire, ou avec un extrait d'un tissu humain, on prélève des cellules spléniques sur un tel animal immunisé, on les fait fusionner avec la lignée cellulaire X63 Ag 8653, on sélectionne les hybridomes et on recueille les anticorps monoclonaux.

4. Procédé selon la revendication 3, caractérisé en ce que la lignée cellulaire humaine cultivée in vitro est une lignée cellulaire permanente.

5. Procédé selon la revendication 3, caractérisé en ce que les mammifères sont des souris.

6. Utilisation d'un anticorps monoclonal selon la revendication 1, dans un produit de diagnostic in vitro.

7. Utilisation d'un anticorps monoclonal selon la revendication 1, en tant que véhicule pour une substance active pharmaceutique.